# EUROPEAN PATENT APPLICATION

(11) **EP 2 997 915 A1**
(43) Date of publication of application: **23.03.2016**
(21) Application number: 14185582.5
(22) Date of filing: 19.09.2014
(51) Int. Cl.: A61B 17/70

(54) **A system for stabilization of at least a portion of a spinal column as well as an anchor member suitable for such a system**

(71) Applicant: Universiteit Maastricht, 6211 LK Maastricht (NL); Academisch Ziekenhuis Maastricht, 6229 HX Maastricht (NL)
(72) Inventor: The designation of the inventor has not yet been filed
(74) Representative: Habets, Winand

(57) **Abstract**

A system for stabilization of at least a portion of a spinal column. The system comprises at least one elongate support member extending along an axis and positionable along the spinal column and at least one anchor member being engageable to a vertebra of the spinal column for attaching the elongate support member to the spinal column. The anchor member comprises a passage configured to receive said support member, allowing substantially unconstrained movement of said elongate support member relative to said anchor member in axial direction parallel to said axis of the elongate support member. At least two elongate support members are attachable to the spinal column by means of said anchor member. The anchor member defines two passages, each configured to receive one of said at least two elongate support members, allowing substantially unconstrained movement of said elongate support members relative to said anchor member.

## Description

### FIELD OF THE INVENTION

The invention relates to a system for stabilization of at least a portion of a spinal column, comprising:
- at least one elongate support member extending along an axis and positionable along the spinal column;
- at least one anchor member being engageable to a vertebra of the spinal column for attaching the elongate support member to the spinal column, which anchor member comprises a passage configured to receive said support member, allowing substantially unconstrained movement of said elongate support member relative to said anchor member in axial direction parallel to said axis of the elongate support member.

The invention also relates to an anchor member suitable for such a system.

### BACKGROUND OF THE INVENTION

Such a system, which is known from EP1871252B1 comprises a number of, for example two, elongate support members, each extending along a longitudinal axis in axial direction. Each elongate support member is engaged across a number of vertebrae levels via a plurality of anchor members. One kind of anchor member is of the fixed or constrained type so as to substantially prevent relative axial movement between the elongate support member and the anchor member, which likewise substantially prevents relative axial movement between the elongate support member and the vertebra to which the anchor member is connected. Another kind of anchor members is of the variable or unconstrained type so as to allow for relative axial movement between the elongate support member and the anchor member. Such variable or unconstrained engagement between the elongate support member and the anchor members likewise allows for relative axial movement between the elongate support member and the vertebrae to which the anchor member is connected, which in turn allows for relative axial movement between each of the vertebrae to accommodate for growth of the patient's spine.

Such systems are used for example in patients with infantile or juvenile scoliosis who undergo curve stabilization.

For each elongate support member a number of anchor members are needed. A disadvantage of this known system is that it is rather difficult to align the elongate support members parallel to each other and that the elongate support members fail to remain in a parallel relationship with respect to each other.

### SUMMARY OF THE INVENTION

The object of the invention is to provide a system for stabilization of at least a portion of the spinal column whereby the parallel orientation of elongate members can easily be obtained and maintained in order to attain a relatively stiff system.

This is accomplished with the system according to the invention in that at least two elongate support members are attachable to the spinal column by means of said anchor member, which anchor member defines two passages, each configured to receive one of said at least two elongate support members, allowing substantially unconstrained movement of said elongate support members relative to said anchor member. The anchor members interconnect the elongate support members which makes the system relatively stiff.

The anchor members keep the, often metal, elongate support members at a distance of the spinal column so that sliding contact between metal elongate support members and the spinal column is prevented. Such a sliding contact may result in irritation of the periosteum, being the membrane that covers the outer surface of bone.

Another embodiment of the system according to the invention is characterized in that said passages extend parallel to each other.

By having parallel passages the anchor member can easily slide with respect to both elongated members, and can easily slide with respect to a number of the same anchor members being spaced apart along the spinal column.

Another embodiment of the system according to the invention is characterized in that said anchor member comprises at least two bushings connected to each other by a bridge part.

Such an anchor element can easily be manufactured, whereby the bushings can be optimized for guiding the elongate support members whilst the bridge part can be optimized for interconnecting the bushings and providing rigidity to the system.

Another embodiment of the system according to the invention is characterized in that said anchor member comprises a first set of two coaxially located bushings spaced apart from a second set of two coaxially located bushings, whilst the bushings in each set are spaced apart.

By having two coaxially located, spaced apart bushings for a single elongate support member, pivoting of the elongate support member with respect to the anchor member is prevented and a relatively rigid system allowing mainly axial movement is obtained.

By having two spaced apart sets of two coaxially located bushings, two elongate support members can be allowed to slide with respect to the same anchor member.

Another embodiment of the system according to the invention is characterized in that the four bushings are connected to each other by a cross-shaped bridge part.

With such a cross-shaped bridge part the four bushings can easily be connected to each other. Furthermore, the cross-shaped bridge part can easily be attached to the spinal column.

Another embodiment of the system according to the invention is characterized in that said anchor member is made of at least a non-metallic polymer, like ultra-high-molecular-weight polyethylene (UHMWPE), poly-ether-ether-ketone (PEEK), polyester, poly carbonate-urethane or other synthetic materials.

Such kind of material has relatively low friction with an elongate support member made from a metal, like titanium, stainless steel, cobalt-chromium, or different alloys so that the elongate support member can easily slide with respect to the anchor member. It is also possible to combine these materials with metal, whereby metal on metal contact as well as contact between metal and the vertebrae is preferably avoided.

Another embodiment of the system according to the invention is characterized in that said anchor member is engageable to a vertebra of the spinal column by means of a sublaminar wire.

Thoracic pedicular anatomy is often distorted in scoliosis patients, which may preclude pedicle screw placement. However, with sublaminar wires the anchor member can easily be attached to such deformed vertebra of the spinal column. Since each anchor member is used for at least two elongate support members, it is still possible to obtain a rigid system. The sublaminar wires can be metallic or polymeric (woven or braided). Examples of polymeric sublaminar wires are UHMWPE or polyester wires. Another embodiment of the system according to the invention is characterized in that said system comprises a device being engageable to the spinal column and to at least one of the at least two elongate support members in a manner that substantially prevents axial movement of said elongate support member relative to said device.

The device has a fixed position with respect to the spinal column at the location where the device is connected to the spinal column. The elongate support member has a fixed position with respect to the device at the location where the elongate support member is connected to the device. In this manner, the device substantially prevents relative axial movement between the elongate support member and the spinal column at the location where the device is connected to the spinal column. The anchor members which are connected to the spinal column at vertebrae at other locations can move with respect to the device by sliding along the elongate support members. The device may comprise pedicle screws, laminar hooks, or may comprise an anchor member as described above wherein the elongate support member is clamped in the passage of the anchor member

### BRIEF DESCRIPTION OF THE DRAWINGS

The system according to the invention will further be explained with reference to the drawings, wherein,
Figure 1 is a perspective view of a system according to the invention,
Figure 2 is a first embodiment of an anchor member according to the invention,
Figure 3 is a second embodiment of an anchor member according to the invention,
Figure 4 is a third embodiment of an anchor member according to the invention,
Figure 5 is a fourth embodiment of an anchor member according to the invention.

In the drawings, like reference numerals refer to like elements.

### DESCRIPTION OF THE FIGURES

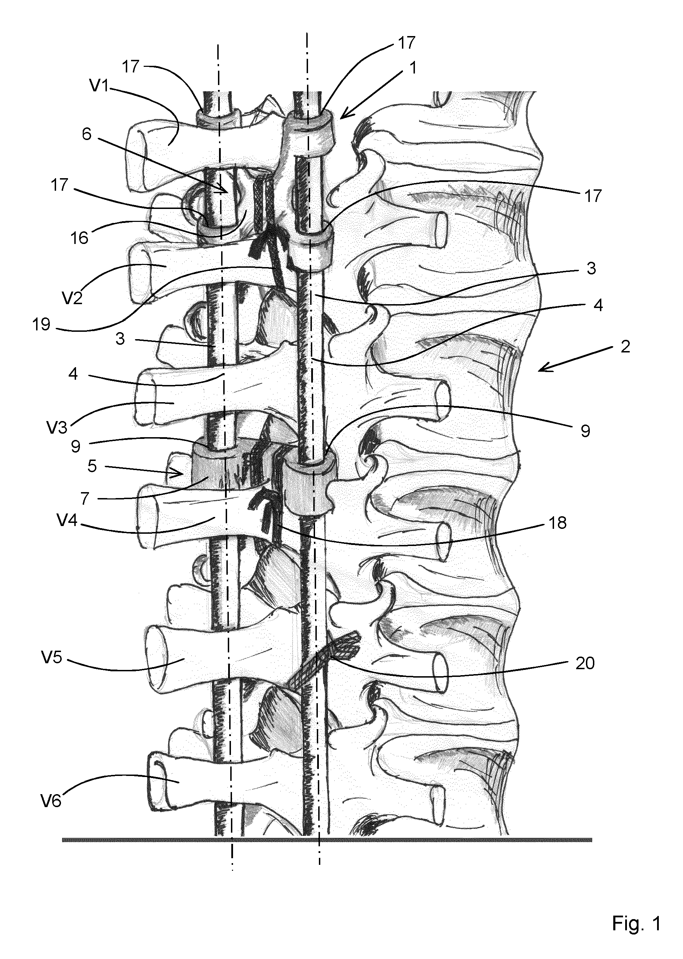

Figure 1 shows a perspective view of a system 1 according to the invention, for stabilization of at least a portion of a spinal column 2. The system 1 comprises two metal elongate support members 3, each extending along an axis 4 as well as anchor member 5, 6. The spinal column 2 comprises a number of vertebrae V1-V6.

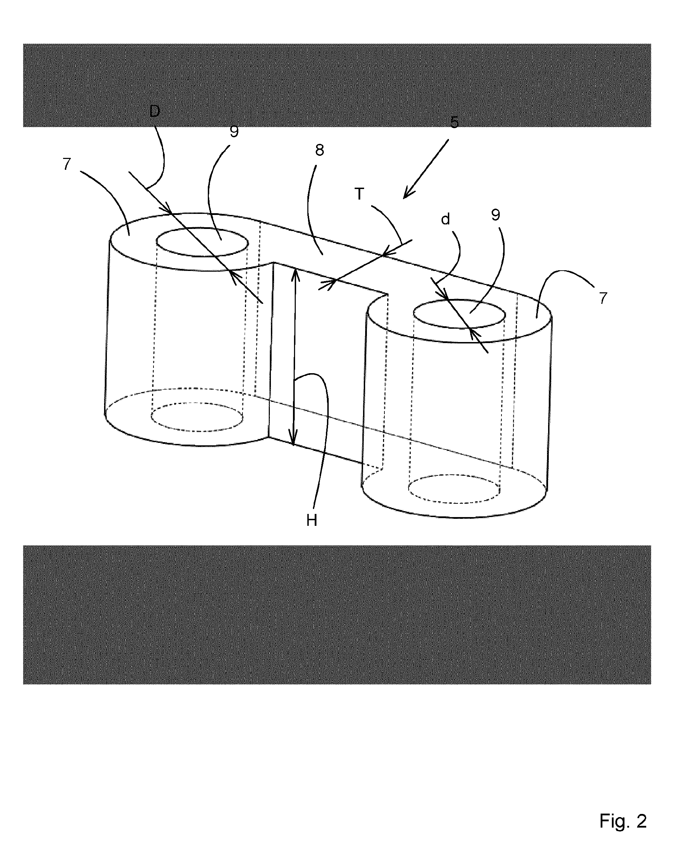

As can be seen in figure 2, the anchor member 5 comprises two bushings 7 connected to each other by a bridge part 8. The two bushings 7 and the bridge part 8 have the same height H. In the embodiment as shown in figure 1 the diameter D of two bushings 7 is larger than the thickness T of the bridge part 8. Each bushing 7 comprises a passage 9 with a diameter d. The passages 9 extend parallel to each other. In an embodiment the height H is in the order of 8-20 millimetre, like 10 millimetre, the diameter D is in the order of 8-20 millimetre, like 10 millimetre, the diameter d is in the order of 3-10 millimetre, like 5 millimetre, the thickness T is in the order of 5-20 millimetre, like 7,5 millimetre and the distance between the two passages 9 is in the order of 15-60 millimetre, like 20 millimetre

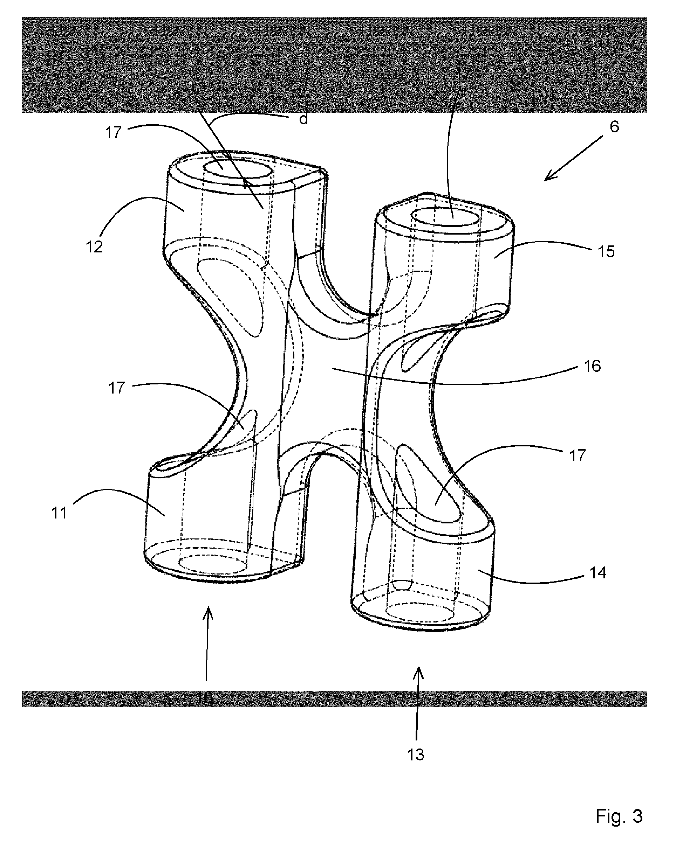

As can be seen in figure 3, the anchor member 6 comprises a first set 10 of two coaxially located bushings 11, 12 spaced apart from a second set 13 of two coaxially located bushings 14, 15. The bushings 11, 12; 14, 15 in each set 10, 13 are spaced apart. The four bushings 11, 12, 14, 15 are connected to each other by a cross-shaped bridge part 16. Each bushing 11, 12, 14, 15 comprises a passage 17. In an embodiment the total height in axial direction is 40 millimetre, the diameter d is 5 millimetre, the distance between two passages 17 of different sets 10, 13 is 20 millimetre and the distance between two passages 17 of the same set 10, 13 is in the order of 30-50 millimetre, like about 20 millimetre

The anchor member 5, 6 is made of a non-metallic polymer, like ultra-high-molecular-weight polyethylene (UHMWPE), poly-ether-ether-ketone (PEEK), polyester, poly carbonate-urethane or other synthetic materials. Each material has a low coefficient of friction with respect to the metal of the elongate support member 3 to allow sliding of the anchor members 5, 6 with respect to the elongate support members 3.

The elongate support member 3 has the same cross section as the passage 9, 17, being slightly smaller to allow sliding movement of the elongate support member 3 with respect to the passage 9, 17. The cross section might be circular as shown in the figures 2, 3. However, the cross section may also be non-circular, thereby preventing that the elongate support member rotates with respect to the passage. Such a non-circular cross section may comprise elliptical, triangular, rectangular, hexagonal or polygonal shapes and configurations.

As can be seen in figure 1, the anchor member 5 is connected to vertebra V4 by means of a sublaminar wire 18,preferably a woven polymeric, for example UHMWPE, sublaminar wire. The sublaminar wire 18 is looped around the vertebra V4 and the bridge part 8. In the same manner the anchor member 6 is connected to vertebra V1 by means of a sublaminar wire 19. The sublaminar wire 19 is looped around the vertebra V1 and the cross-shaped bridge part 16. The sublaminar wire 19 may also be looped around the elongate support members 3.

Before attaching the anchor members 5, 6 to the vertebrae V4, V1 the elongate support members 3 have been inserted through the passages 9, 17 of the anchor members 5, 6. So, when the anchor members 5, 6 are engaged to the vertebrae V1, V4 of the spinal column 2, the elongate support members 3 are attached to the spinal column 2 in a parallel relationship, whereby the elongate support members 3 and the anchor members 5, 6 form a rigid system 1 for the spinal column 2. In case of elongate support members 3 have non-circular cross sections, only substantially unconstrained movement of the elongate support members 3 relative to the anchor members 5, 6 and the vertebrae V1-V6 in axial direction parallel to the axes 4 of the elongate support members 3 is possible in case of a growth of the spinal column 2, whereby the distances between the vertebrae V4 and V1 with the anchor members 5, 6 will get larger. No other movements of the elongate support members 3 relative to the anchor members 5, 6 are possible.

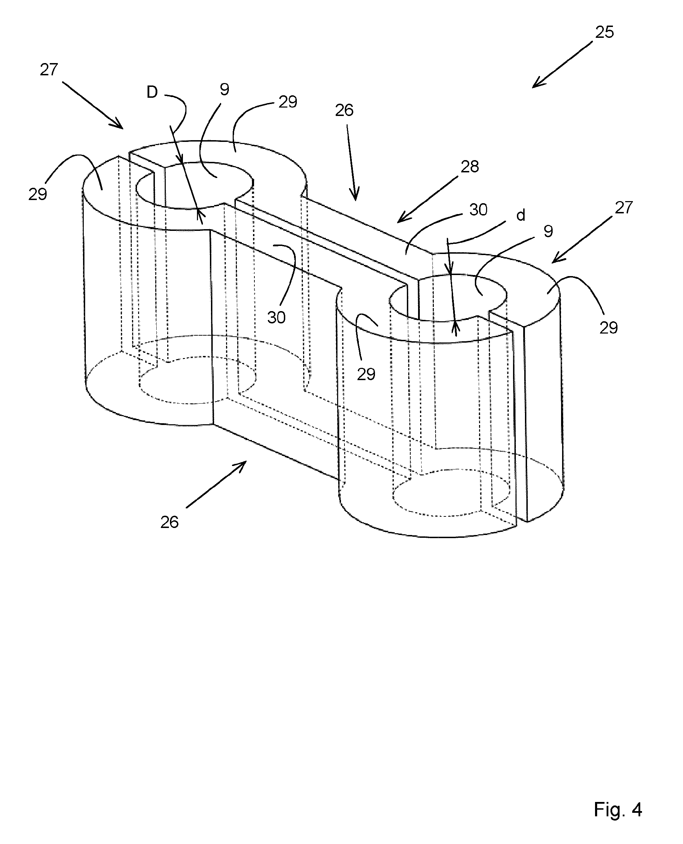

Figure 4 shows a third embodiment of an anchor member 25 according to the invention, which anchor member 25 comprises two identical halves 26 positioned opposite to each other to form two bushings 27 connected to each other by a bridge part 28. Each bushing 27 comprises a passage 9 with a diameter d. The passages 9 extend parallel to each other. The anchor member 25 can have similar dimensions as the anchor member 5 and be made of the same material. Each half 26 comprises two half-cylindrical parts 29 of the bushings 27 connected to each other by half 30 of the bridge part 28. The halves 26 can be connected to each other by a glue or by mechanical means like screws or wires. The halves 26 can be connected to each other before positioning the elongate support members 3 inside the passages 9. However, it is also possible to firstly position each elongate support member 3 to a half-cylindrical part 29 of the bushing 27 of a first half 26 and then connect the other half 26 to the first half 26, thereby enclosing the elongate support member 3. If desired, the first half 26 can be connected to a vertebra V1-V6 before connecting the second half 26 to the first half 26. The halves 26 can also have different shapes as long as the combined halves 26 define the desired passages.

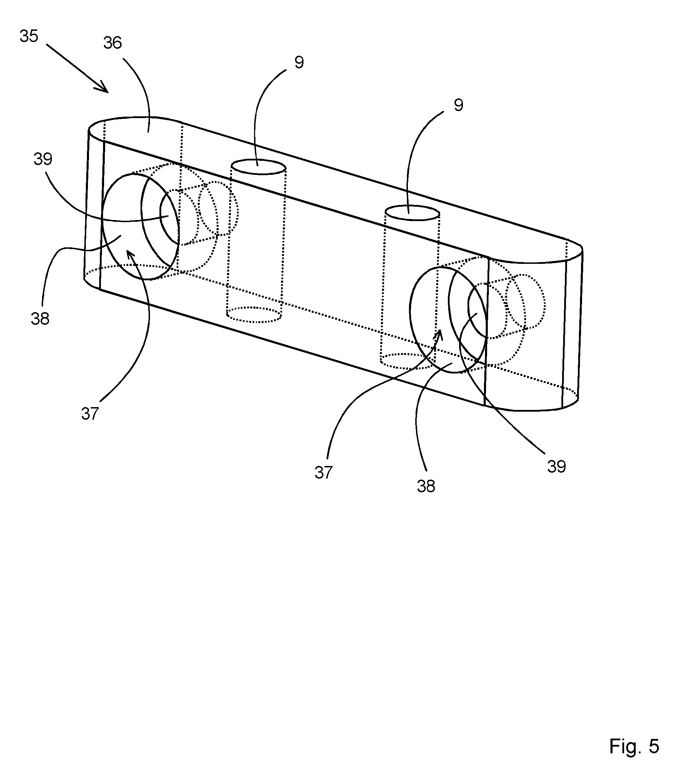

Figure 5 shows a fourth embodiment of an anchor member 35 according to the invention, which anchor member 35 comprises a body 36 provided with two spaced apart passages 9 and two spaced apart passages 37. The passages 9 extend parallel to each other and are suited for guiding the elongate support members 3.

The passages 37 extend parallel to each other and perpendicular to the passages 9. The passages 37 comprise a first part 38 having a larger diameter than a second coaxial part 39. Through the passages 37 pedicle screws can be inserted to connect the anchor member 35 to a vertebra V1-V6. A head of a pedicle screw having a larger diameter is being located in the first part 38, whilst a threaded part of the pedicle screw having a smaller diameter is guided through the second part 39 and into the vertebra V1-V6.

The system 1 may be provided with means to avoid that the elongate support members 3 slide out of all the anchor members 5, 6, 25. This means may be formed by thickened or curved end parts of the elongate support members 3. It can also be formed by means of a device, such as like a pedicle screw which is attached to a vertebrae, whereby the elongate support member is connected to the pedicle screw in a manner that substantially prevents axial movement of said elongate support member relative to the pedicle screw.

In the system 1 as shown in figure 1 two different anchor members 5, 6 are shown. However, it is also possible to only use a number of the same kind of anchor members 5, 6 in a system 1.

The system 1 may comprise more than two elongate support members 3, whereby the anchor member 5, 6, 25 comprises the corresponding number of passages 9 17.

The elongate support members 3 can be made of metal like titanium, cobalt-chromium, stainless steel or different metal alloys, but can also be made from any other kind of material.

It is also possible to connect the anchor members 5, 6, 25 by means of pedicle screws to the vertebrae, or a combination of sublaminar wires and pedicle screws.

The person skilled in the art will realize that the present invention is by no means limited to the preferred embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the scope should not be construed as limiting the scope of the claims.

### LIST OF REFERENCE SIGNS

- 1.: system
- 2.: spinal column
- 3.: support member
- 4.: axis
- 5.: anchor member
- 6.: anchor member
- 7.: bushing
- 8.: bridge part
- 9.: passage
- 10.: set
- 11.: bushing
- 12.: bushing
- 13.: set
- 14.: bushing
- 15.: bushing
- 16.: bridge part
- 17.: passage
- 18.: wire
- 19.: wire
- 25: anchor member
- 26: half
- 27: bushing
- 28: bridge part
- 29: part
- 30: half
- 35: anchor member
- 36: body
- 37: passage
- 38: first part
- 39: second part
- D: diameter
- d: diameter
- H: height
- T: thickness
- V1: vertebra
- V2: vertebra
- V3: vertebra
- V4: vertebra
- V5: vertebra
- V6: vertebra

## Claims

1. A system (1) for stabilization of at least a portion of a spinal column (2), comprising:
- at least one elongate support member (3) extending along an axis (4) and positionable along the spinal column (2);
- at least one anchor member (5, 6, 25, 35) being engageable to a vertebra (V1-V6) of the spinal column (2) for attaching the elongate support member (3) to the spinal column (2), which anchor member (5, 6, 25, 35) comprises a passage (9, 17) configured to receive said support member (3), allowing substantially unconstrained movement of said elongate support member (3) relative to said anchor member (5, 6, 25, 35) in axial direction parallel to said axis (4) of the elongate support member (3), **characterized in that** at least two elongate support members (3) are attachable to the spinal column (2) by means of said anchor member (5, 6, 25, 35), which anchor member (5, 6, 25, 35) defines two passages (9, 17), each configured to receive one of said at least two elongate support members (3), allowing substantially unconstrained movement of said elongate support members (3) relative to said anchor member (5, 6, 25, 35).

2. A system (1) according to claim 1, **characterized in that** said passages (9, 17) extend parallel to each other.

3. A system (1) according to claim 1 or 2, **characterized in that** said anchor member (5, 6, 25, 35) comprises at least two bushings (7, 11, 12, 14, 15) connected to each other by a bridge part (8, 16).

4. A system (1) according to claim 3, **characterized in that** said anchor member (5, 6, 25, 35) comprises a first set (10) of two coaxially located bushings (11, 12) spaced apart from a second set (13) of two coaxially located bushings (14, 15), whilst the bushings (11, 12; 14, 15) in each set (10, 13) are spaced apart.

5. A system (1) according to claim 4, **characterized in that** the four bushings (11, 12; 14, 15) are connected to each other by a cross-shaped bridge part (16).

6. A system (1) according to one of the preceding claims, **characterized in that** said anchor member (5, 6, 25, 35) is made of at least a non-metallic polymer, like ultra-high-molecular-weight polyethylene (UHMWPE), poly-ether-ether-ketone (PEEK), polyester, poly carbonate-urethane or other synthetic materials.

7. A system (1) according to one of the preceding claims, **characterized in that** said anchor member (5, 6, 25, 35) is engageable to a vertebra (V1-V6) of the spinal column (2) by means of a sublaminar wire (18, 19), or a pedicle screw.

8. A system (1) according to one of the preceding claims, **characterized in that** said system (1) comprises a device being engageable to the spinal column (2) and to at least one of the at least two elongate support members (3) in a manner that substantially prevents axial movement of said elongate support member (3) relative to said device.

9. An anchor member (5, 6, 25, 35) suitable for a system (1) according to one of the preceding claims, **characterized in that** the anchor member (5, 6, 25, 35) comprises two bushings (7, 11, 12, 14, 15) connected to each other by a bridge part (8, 16).

10. An anchor member (5, 6, 25, 35) according to claim 9, **characterized in that** said anchor member (5, 6, 25, 35) comprises a first set (10) of two coaxially located bushings (11, 12) spaced apart from a second set (13) of two coaxially located bushings (14, 15), whilst the bushings (11, 12; 14, 15) in each set (10, 13) are spaced apart.

11. An anchor member (5, 6, 25, 35) according to claim 10, **characterized in that** the four bushings (11, 12; 14, 15) are connected to each other by a cross-shaped bridge part (16).

12. An anchor member (5, 6, 25, 35) according to one of the preceding claims 9-11, **characterized in that** said anchor member (5, 6, 25, 35) is made of at least a non-metallic polymer, like Ultra-high-molecular-weight polyethylene (UHMWPE), poly-ether-ether-ketone (PEEK), polyester, poly carbonate-urethane or other synthetic materials.
